# EUROPÄISCHE PATENTSCHRIFT

(11) **EP 2 593 163 B1**
(45) Veröffentlichungstag und Bekanntmachung des Hinweises auf die Patenterteilung: **12.08.2015**
(21) Anmeldenummer: 11725772.5
(22) Anmeldetag: 20.06.2011
(51) Int. Cl.: A61M 5/315

(54) **SPRITZE MIT VERMINDERTER HAFTREIBUNG**
SYRINGE HAVING REDUCED STATIC FRICTION
SERINGUE À FROTTEMENT PAR ADHÉRENCE RÉDUIT

(30) Priorität: 15.07.2010 DE 102010027243
(43) Veröffentlichungstag der Anmeldung: 22.05.2013
(73) Patentinhaber: Transcodent GmbH & Co. KG, 24149 Kiel (DE)
(72) Erfinder: HEINZ, Jochen, 24220 Flintbek (DE)
(74) Vertreter: Glawe, Delfs, Moll
(86) Internationale Anmeldenummer: PCT/EP2011/060189
(87) Internationale Veröffentlichungsnummer: WO 2012/007251

(56) Entgegenhaltungen:
- EP-A1- 1 264 611
- EP-A1- 2 016 962
- WO-A1-2007/116086
- GB-A- 1 168 201
- GB-A- 1 260 103

## Beschreibung

Die Erfindung betrifft eine Spritze mit einem Zylinder und einem in dem Zylinder angeordneten Kolben. Es ist ein Dichtelement vorgesehen, das mit einem Kontaktbereich an einer Innenwand des Zylinders anliegt und einen Ringspalt zwischen dem Kolben und einer Innenwand des Zylinders abdichtet. Der Kontaktbereich weist einen Frontabschnitt auf, der im Ruhezustand mit einem Anlagedruck an der Innenwand des Zylinders anliegt. Der Kolben ist so mit dem Dichtelement verbunden, dass das Dichtelement einer Anfangsbewegung des Kolbens aus dem Ruhezustand heraus nicht folgt und dass das Dichtelement einer fortgesetzten Bewegung des Kolbens folgt.

Solche Spritzen dienen dazu, einen flüssigen oder pastösen Stoff gezielt und dosiert auszubringen. Zum Einsatz kommen solche Spritzen beispielsweise bei medizinischen und zahnmedizinischen Anwendungen. Der Stoff ist in einem Innenraum der Spritze enthalten. Durch Druck auf den Kolben dringt der Kolben weiter in den Zylinder ein, so dass der für den Stoff zur Verfügung stehende Raum kleiner wird. Der Stoff tritt aus dem Zylinder aus und wird - zum Beispiel durch eine Hohlnadel hindurch - an der gewünschte Stelle abgegeben.

Mit dem Dichtelement wird der Innenraum des Zylinders abgedichtet, so dass der Stoff daran gehindert ist, zwischen dem Kolben und der Innenwand des Zylinders hindurch auszutreten. Um eine hinreichende Dichtwirkung zu haben, liegt das Dichtelement mit einem Anlagedruck an der Innenwand des Zylinders an. Wenn die Spritze im Ruhezustand ist, besteht durch den Anlagedruck eine Haftreibung zwischen dem Dichtelement und der Innenwand des Zylinders. Die Haftreibung zwischen zwei relativ zueinander ruhenden Flächen ist bekanntlich größer als die Gleitreibung zwischen zwei Flächen, die sich relativ zueinander bewegen. Bei der Bedienung der Spritze muss zunächst die Haftreibung überwunden werden, um das Dichtelement relativ zu dem Zylinder in Bewegung zu setzen. Es besteht also ein vergleichsweise hoher Anfangswiderstand, bevor sich das Dichtelement bewegt. Ein hoher Anfangswiderstand bei der Bedienung der Spritze ist zum einen wenig bedienungsfreundlich. Zum anderen kann ein plötzliches Losbrechen des Kolbens dazu führen, dass der Inhalt der Spritze unkontrolliert austritt.

Der Erfindung liegt ausgehend von diesem Stand der Technik die Aufgabe zu Grunde, eine Spritze mit verbesserter Bedienungsfreundlichkeit vorzustellen. Die Aufgabe wird gelöst durch die Merkmale des unabhängigen Anspruchs. Erfindungsgemäß wird bei einer Anfangsbewegung des Kolbens der Anlagedruck zwischen dem Frontabschnitt des Kontaktbereichs und der Innenwand des Zylinders vermindert. Vorteilhafte Ausführungsformen finden sich in den Unteransprüchen.

Zunächst werden einige Begriffe erläutert. Wenn die Spritze im Ruhezustand ist, bewegt sich der Kolben nicht relativ zu dem Zylinder. Das Dichtelement liegt über den Umfang des Kolbens an der Innenwand des Zylinders an, wobei ein hinreichender Anlagedruck besteht, so dass der Innenraum der Spritze abgedichtet ist. Aus dem Ruhezustand heraus kann der Kolben eine Anfangsbewegung durchführen, ohne dass das Dichtelement der Anfangsbewegung folgt.

Der Kontaktbereich zwischen dem Dichtelement und der Innenwand des Zylinders hat in Längsrichtung der Spritze betrachtet eine Ausdehnung, die sich von einem hinteren Ende bis zu einem vorderen Ende erstreckt. Das vordere Ende weist in die Richtung, in die der Stoff aus der Spritze austritt, also zum Auslassende. Das hintere Ende weist in die Richtung, von dem aus die Spritze bedient wird, an dem also bei der Bedienung der Spritze Druck auf den Kolben ausgeübt wird. Der Begriff Frontabschnitt des Kontaktbereichs bezeichnet das vordere Ende sowie den sich unmittelbar daran anschließenden Abschnitt des Kontaktbereichs.

Der Begriff Anfangsbewegung bezieht sich auf eine Bewegung des Kolbens relativ zu dem Zylinder und bezeichnet eine Bewegung in Richtung Auslassende der Spritze, die aus dem Ruhezustand heraus stattfindet. Eine fortgesetzte Bewegung schließt sich an eine Anfangsbewegung an.

Wenn der Anlagedruck zwischen dem Dichtelement und der Innenwand des Zylinders vermindert wird, so geht dies in aller Regel damit einher, dass ein Frontabschnitt des Kontaktbereichs von der Innenwand abgehoben wird. Im Frontabschnitt des Kontaktbereichs besteht dann kein Anlagedruck mehr zwischen dem Dichtelement und der Innenwand. In einem Extremfall können die aufeinander liegenden Flächenbereiche auch unverändert bleiben und sich lediglich der Anlagedruck im Frontabschnitt des Kontaktbereichs auf einen von null verschiedenen Wert vermindern. In einem anderen Extremfall wird der Kontaktbereich vollständig von der Innenwand abgehoben , was zwangsläufig damit einhergeht, dass auch im Frontabschnitt des Kontaktbereichs kein Anlagedruck mehr besteht. Dies kommt insbesondere dann in Betracht, wenn das Dichtelement mehrere voneinander separate Kontaktbereiche aufweist.

Die Erfindung hat erkannt, dass die Haftreibung, die überwunden werden muss, um das Dichtelement aus dem Ruhezustand heraus gegenüber dem Zylinder in Bewegung zu setzen, zu einem wesentlichen Teil aus dem Anlagedruck im Frontabschnitt des Kontaktbereichs resultiert. Wenn man den Anlagedruck im Frontabschnitt des Kontaktbereichs reduziert, wird dadurch die Haftreibung zwischen dem Dichtelement und der Innenwand des Zylinders deutlich vermindert und das Dichtelement lässt sich leichter in Bewegung versetzen.

Erfindungsgemäß wird vorgeschlagen, eine Anfangsbewegung, die zwischen dem Kolben und dem Zylinder stattfinden kann, ohne dass sich zugleich das Dichtelement bewegt, auszunutzen, um den Anlagedruck im Frontabschnitt des Kontaktbereichs zu vermindern. Durch die Anfangsbewegung wird also die Kraftübertragung zwischen dem Kolben und dem vorderen Kontaktbereich so verändert, dass der Frontabschnitt des Kontaktbereichs mit einem geringeren Druck an der Innenwand des Zylinders anliegt.

Im Ruhezustand ist die Spritze auch über einen längeren Zeitraum dicht, was dadurch erreicht wird, dass das Dichtelement mit einem hinreichenden Anlagedruck an der Innenwand des Zylinders anliegt. Zwar mag der Anlagedruck nach der Anfangsbewegung nicht mehr hinreichend in diesem Sinne sein, also nicht mehr ausreichen, um die Spritze auf Dauer dichtzuhalten. Dies kann aber in Kauf genommen werden, da es sich nur um einen kurzen Zeitraum handelt und das Dichtelement ohne weiteres so gestaltet werden kann, dass der Stoff trotzdem sicher aus der Spritze ausgebracht werden kann. Durch den verminderten Anlagedruck im Frontabschnitt des Kontaktbereichs unterscheidet sich die Erfindung von Spritzen, wie sie in DE 10 2007 034 477 A1 offenbart sind.

GB 1260103 offenbart den Oberbegriff Anspruch 1.

Mit der Anfangsbewegung bewegt sich der Kolben in dem Zylinder nach vorne, also in Längsrichtung der Spritze. Der Anlagedruck im Frontabschnitt des Kontaktbereichs, der mit der Anfangsbewegung vermindert werden soll, hingegen wirkt in Radialrichtung. Die Bewegung in Längsrichtung muss folglich so umgesetzt werden, dass sie eine veränderte Kraftübertragung in Radialrichtung zur Folge hat. Für die konkrete Umsetzung dieser Kraftübertragung gibt es mehrere Möglichkeiten, von denen einige nachfolgend erläutert werden.

Das Dichtelement kann so gestaltet sein, dass es ohne zusätzliche Einwirkung von außen einen hinreichenden Anlagedruck gegenüber der Innenwand des Zylinders aufbringt. Dies kann beispielsweise dadurch erreicht werden, dass das Dichtelement in entspanntem Zustand einen etwas größeren Durchmesser hat als der Zylinder, so dass es leicht komprimiert werden muss, um in den Zylinder eingeführt werden zu können. Mit der Erfindung kann ein Übertragungselement zwischen dem Kolben und dem Dichtelement vorgesehen sein, mit dem bei der Anfangsbewegung des Kolbens eine Kraft auf das Dichtelement übertragen wird, um den Anlagedruck zu vermindern. Insbesondere kann das Übertragungselement so ausgebildet sein, dass es eine Zugkraft auf das Dichtelement überträgt. Für eine wirksame Übertragung der Zugkraft kann sich das Übertragungselement vom vorderen Kontaktbereich des Dichtelements aus schräg nach vorne in Richtung des Kolbens erstrecken.

Die erfindungsgemäße Spritze ist vorzugsweise so gestaltet, dass mit der Verminderung des Anlagedrucks eine deutliche Verminderung der Haftreibung zwischen dem Dichtelement und der Innenwand bewirkt wird. Beispielsweise kann die Haftreibung zwischen dem Kontaktbereich und der Innenwand nach der Anfangsbewegung um mindestens 10%, vorzugsweise um mindestens 20%, weiter vorzugsweise um mindestens 30% geringer sein als im Ruhezustand. Geht man davon aus, dass die Haftreibung im Allgemeinen um etwa 50% bis 100% größer ist als die Gleitreibung, ist bei der erfindungsgemäßen Spritze die Haftreibung nicht mehr wesentlich größer, als es die Gleitreibung ohne die erfindungsgemäße Verminderung des Anlagedrucks wäre. Wenn mehrere Kontaktbereiche vorgesehen sind, reduziert sich vorzugsweise die Haftreibung des gesamten Dichtelements um mindestens 10%, vorzugsweise mindestens 20%, weiter vorzugsweise mindestens 30%.

Das Dichtelement kann auch so gestaltet sein, dass es nicht von sich aus einen ausreichenden Anlagedruck gegenüber der Innenwand des Zylinders hat, sondern dass der Anlagedruck durch äußere Einwirkung erzeugt wird. Dazu kann der Kolben so gestaltet sein, dass er im Ruhezustand eine radial nach außen wirkende Kraft auf das Dichtelement ausübt. Mit der Anfangsbewegung des Kolbens vermindert sich die radial nach außen wirkende Kraft. Beispielsweise kann der Kolben dazu so gestaltet sein, dass er einen konischen Abschnitt aufweist, so dass das Dichtelement im Ruhezustand auf dem dickeren Ende des Konus und nach der Anfangsbewegung auf dem dünneren Ende des Konus sitzt.

Um dem Dichtelement die für die Verminderung des Anlagedrucks erforderliche Bewegungsfreiheit zu geben, kann im Ruhezustand ein Freiraum zwischen dem Dichtelement und dem Kolben bestehen. In den Freiraum kann sich das Dichtelement bei der Anfangsbewegung des Kolbens bewegen. Vorzugsweise besteht im Ruhezustand ein Freiraum insbesondere in dem Bereich, der von dem Frontabschnitt des Kontaktbereichs aus radial innen liegt.

Mit seiner Anfangsbewegung bewegt sich der Kolben ohne das Dichtelement, das Dichtelement bleibt also zunächst in der Position, die es im Ruhezustand hat. Die Spritze kann so gestaltet sein, dass die Position des Dichtelements in dieser Phase alleine durch die Haftreibung zwischen dem Dichtelement und der Innenwand des Zylinders bestimmt ist. Alternativ kann eine Hülse in dem Ringspalt zwischen dem Kolben und dem Zylinder vorgesehen sein, die das Dichtelement führt. Mit einer solchen Hülse ist die Führung des Dichtelements präziser, als wenn das Dichtelement alleine durch die Haftreibung in seiner Position gehalten wird. Die Hülse ist vorzugsweise so ausgebildet, dass sie sich während der Anfangsbewegung des Kolbens nicht bewegt. Einer fortgesetzten Bewegung des Kolbens kann die Hülse folgen und sich gleichzeitig mit dem Dichtelement in Bewegung setzen. Die Hülse kann sich in Längsrichtung der Spritze von dem Dichtelement bis zum hinteren Ende des Zylinders oder weiter bis zum hinteren Ende des Kolbens erstrecken.

Die Anfangsbewegung, mit der der Anlagedruck im Frontabschnitt des Kontaktbereichs vermindert wird, führt der Kolben durch, wenn Druck auf eine Druckfläche am hinteren Ende des Kolbens ausgeübt wird. In einer vorteilhaften Ausführungsform ist die Spritze so gestaltet, dass sie sich von selbst wieder in die Ruheposition zurück bewegt, sobald kein Druck mehr auf die Druckfläche ausgeübt wird. Dies kann erreicht werden, indem mit der Anfangsbewegung ein elastisches Element gespannt wird, das nach dem Wegfall des Drucks wieder in seine ursprüngliche Position zurückkehrt und damit den Kolben in die Ruheposition bewegt.

Die Spritze kann so ausgebildet sein, dass das Übertragungselement und/oder das Dichtelement selbst zugleich als elastisches Element wirken, das mit der Anfangsbewegung gespannt wird. Das elastische Element kann auch ein separates Element sein. Wenn eine Hülse zum Führen des Dichtelements vorgesehen ist, kann das elastische Element mit seinem einen Ende an dem Kolben und mit seinem anderen Ende an der Hülse angreifen.

Das Dichtelement kann so gestaltet sein, dass es einen einzelnen einheitlichen Kontaktbereich aufweist. Das Dichtelement kann auch mehrere voneinander getrennte Kontaktbereiche aufweisen, beispielsweise in Form mehrerer Dichtlippen, die hintereinander angeordnet sind. An das hintere Ende des vorderen Kontaktbereichs schließt sich dann ein Freibereich an, in dem das Dichtelement keinen Kontakt mit der Innenwand des Zylinders hat. Der Freibereich geht über in den Frontabschnitt des nächsten Kontaktbereichs. Insbesondere wenn das Dichtelement mehrere Kontaktbereiche umfasst, kann das Vermindern des Anlagedrucks auch darin bestehen, dass einer der Kontaktbereiche vollständig von der Innenwand des Zylinders abgehoben wird. Mit dem vollständigen Abheben geht zwangsläufig eine Verminderung des Anlagedrucks im Frontabschnitt des Kontaktbereichs einher. Wenn ein Kontaktbereich vollständig abgehoben wird, so ist dies vorzugsweise ein hinterer Kontaktbereich, da ansonsten die in der Spritze enthaltene Substanz in den Bereich des Stopfens eintreten kann.

Eine bevorzugte Anwendung der erfindungsgemäßen Spritze besteht darin, dass im Rahmen einer Zahnbehandlung Dentalmassen ausgebracht werden. Ein weiterer medizinischer Anwendungsbereich ist die Injektion von Wirkstoffen in den menschlichen Körper. Nicht ausgeschlossen ist es, dass erfindungsgemäße Spritzen auch beim Basteln oder Heimwerkern Verwendung finden können.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine schematische Darstellung einer erfindungsgemäßen Spritze;
- Fig. 2 bis 5:: Detailansichten verschiedener erfindungsgemäßer Ausführungsformen;
- Fig. 6:: zwei Darstellungen einer weiteren Ausführungsform einer erfindungsgemäßen Spritze;
- Fig. 7:: die Ansicht aus Fig. 6 bei einer weiteren Ausführungsform der Erfindung; und
- Fig. 8:: Detailansichten aus Fig. 7.

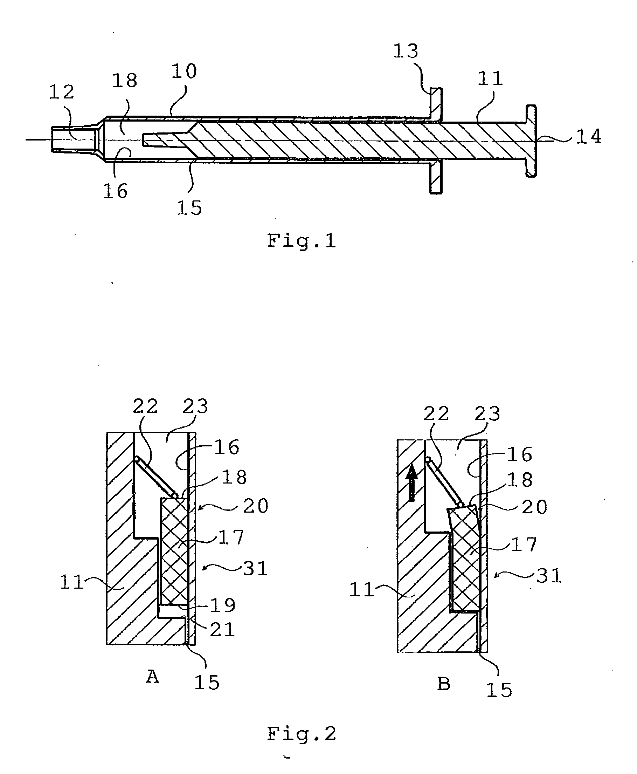

Eine in Fig. 1 gezeigte Spritze umfasst einen Spritzenkörper in Form eines Zylinders 10, in dem ein Kolben 11 aufgenommen ist. Am vorderen Ende des Zylinders 10 ist ein Auslass 12 ausgebildet, durch den ein in der Spritze enthaltener Stoff austreten kann. An seinem hinteren Ende weist der Zylinder 10 einen Kragen 13 auf, der gegenüber der Wand des Zylinders 10 nach außen vorspringt. Das hintere Ende des Kolbens 11 ist als Druckfläche 14 ausgebildet, über die Druck auf den Kolben 11 ausgeübt werden kann, um den Kolben 11 relativ zu dem Zylinder 10 in Bewegung zu setzen. Wenn eine Bedienperson Druck auf die Druckfläche 14 ausübt, kann sie den Kragen 13 als Gegenlager nutzen.

Der durch den Zylinder 10 und den Kolben 11 umschlossene Innenraum 23 der Spritze kann mit einem flüssigen oder pastösen Stoff gefüllt werden, der durch den Auslass 12 gezielt und dosiert ausgebracht werden soll. Wird nun durch Druck auf die Druckfläche 14 der Kolben 11 relativ zu dem Zylinder 10 in Bewegung gesetzt, verkleinert sich das Volumen des Innenraums der Spritze und es tritt Material nach vorne durch den Auslass 12 aus.

Um zu verhindern, dass das Material durch den Ringspalt 15 zwischen dem Kolben 11 und der Innenwand 16 des Zylinders 10 nach hinten austreten kann, ist ein in Fig. 1 nicht gezeigtes Dichtelement vorgesehen, das sich um den Kolben 11 herum erstreckt und den Ringspalt 15 abdichtet. Das Dichtelement liegt mit einem als Anlagedruck bezeichneten Druck an der Innenfläche 16 des Zylinders 10 an und dichtet diesen Austrittspfad ab. Um den Kolben 11 zusammen mit dem Dichtelement in Bewegung zu versetzen, muss die zwischen dem Dichtelement und der Innenwand 16 des Zylinders 10 bestehende Haftreibung überwunden werden. Dies erfordert bei bisherigen Spritzen einen erhöhten Druck auf die Druckfläche 14 des Kolbens 11, was als wenig bedienungsfreundlich empfunden wird. Mit der erfindungsgemäßen Spritze wird die Kraft, die erforderlich ist, um das Dichtelement relativ zu dem Zylinder 10 in Bewegung zu setzen, vermindert. Es wird dadurch möglich, den Kolben 11 langsam und gleichmäßig in Bewegung zu setzen, so dass auch der Stoff langsam und gleichmäßig aus der Spritze austritt. Für einen Patienten, dem ein Wirkstoff injiziert wird, ist dies angenehmer als ein ruckartiges Losbrechen des Kolbens 11, mit dem eine unkontrollierte Menge ausgebracht wird. Letzteres kann dem Patienten Schmerzen verursachen.

In Fig. 2 ist ein Ausschnitt einer ersten erfindungsgemäßen Ausführungsform gezeigt. In Abbildung A befindet sich die Spritze im Ruhezustand. Der Innenraum 23 ist mit dem auszubringenden Stoff gefüllt. Der Kolben 11 bewegt sich nicht relativ zu dem Zylinder 10 und auf die Druckfläche 14 des Kolbens 11 wird kein Druck ausgeübt. Ein Dichtelement 17 erstreckt sich um den Kolben 11 herum und dichtet den Ringspalt 15 zwischen dem Kolben 11 und dem Zylinder 10 ab. Mit seiner äußeren Mantelfläche liegt das Dichtelement 17 mit einem hinreichenden Anlagedruck an der Innenwand 16 des Zylinders 10 an, so dass der Stoff auch bei dauerhafter Lagerung nicht aus der Spritze austreten kann. Der hinreichende Anlagedruck ergibt sich aus der Form des Dichtelements 17. Die Mantelfläche des Dichtelements 17 liegt flächig auf der Innenwand 16 des Zylinders auf, so dass die Mantelfläche insgesamt den Kontaktbereich 31 zwischen dem Dichtelement 17 und der Innenwand 16 des Zylinders 10 bildet. Der Kontaktbereich 31 erstreckt sich von einem vorderen Ende 18 bis zu einem hinteren Ende 19. Der Frontabschnitt 20 des Kontaktbereichs 31 schließt sich unmittelbar an das vordere Ende 18 an. Bewegt man sich ausgehend vom Frontabschnitt 20 des Kontaktbereichs 31 weiter in Richtung des vorderen Endes der Spritze, so besteht dort kein Kontakt mehr zwischen dem Dichtelement 17 und der Innenwand 16 des Zylinders 10.

Der Kolben 11 ist so in dem Zylinder 10 gelagert, dass er sich aus dem Ruhezustand heraus in Form einer Anfangsbewegung ein kleines Stück in Richtung des vorderen Endes der Spritze bewegen kann, ohne dass das Dichtelement 17 dieser Bewegung folgt. Erst wenn zum Ende der Anfangsbewegung eine Schulter 21 am hinteren Ende 19 des Dichtelements 17 anstößt (siehe Ansicht B), wird das Dichtelement 17 gezwungen, einer fortgesetzten Bewegung des Kolbens 11 zu folgen.

Zwischen dem vorderen Ende 18 des Dichtelements 17 und dem Kolben 11 erstreckt sich ein Übertragungselement 22. Das Übertragungselement 22 schließt mit der Längsachse der Spritze einen Winkel von etwa 45° an und ist mit dem Dichtelement 17 sowie mit dem Kolben 11 gelenkig verbunden. Mit der Anfangsbewegung des Kolbens 11 wird das Übertragungselement 22 nach vorne gezogen und übt damit eine Zugkraft auf das vordere Ende 18 des Dichtelements 17 aus. Das vordere Ende 18 des Dichtelements 17 wird dadurch nach innen gezogen, so dass das Dichtelement 17 im Frontabschnitt 20 des Kontaktbereichs 31 von der Innenwand 16 des Zylinders 10 abgehoben wird. Der Anlagedruck im Frontabschnitt 20 des Kontaktbereichs 31 reduziert sich auf null und ist damit kleiner als im Ruhezustand. Mit einer Mehrzahl von über den Umfang des Kolbens 11 verteilten Übertragungselementen 22 kann der Frontabschnitt 20 des Kontaktbereichs 31 über den gesamten Umfang des Dichtelements 17 von der Innenwand 16 abgehoben werden.

Wenn das Dichtelement 17 mit dem Frontabschnitt 20 des Kontaktbereichs 31 von der Innenwand 16 des Zylinders 10 abgehoben ist, ist es deutlich leichter, die Haftreibung zwischen dem Dichtelement 17 und der Innenwand 16 zu überwinden. Insbesondere ist die Gefahr vermindert, dass sich das Dichtelement 17 gegenüber der Innenwand 16 verkantet und sich die zum Losbrechen erforderliche Kraft dadurch noch weiter erhöht. Indem das Dichtelement 17 leichter in Bewegung versetzt werden kann, ergibt sich insgesamt ein sehr gleichmäßiger Kraftaufbau, wenn aus dem Ruhezustand heraus Druck auf die Druckfläche 14 des Kolbens 11 aufgebaut wird. Zunächst lässt sich der Kolben 11 leicht in Bewegung versetzen. Zunehmend wird über das Übertragungselement 22 ein Gegenzug aufgebaut, bis die Schulter 21 am hinteren Ende 19 des Dichtelements 17 anliegt. Dann kann die nunmehr verminderte Haftreibung zwischen dem Dichtelement 17 und der Innenwand 16 überwunden werden.

Wird kein weiterer Druck mehr auf die Druckfläche 14 des Kolbens 11 ausgeübt, kommt die Bewegung des Kolbens 11 zum Stillstand. Das mit der Anfangsbewegung elastisch verformte Dichtelement 17 kehrt in seinen ursprünglichen Zustand zurück und zieht über das Übertragungselement 22 den Kolben 11 wieder ein Stück nach hinten. Die Spritze befindet sich damit wieder im Ruhezustand, aus dem heraus der geschilderte Ablauf bei Bedarf wieder von vorne beginnen kann.

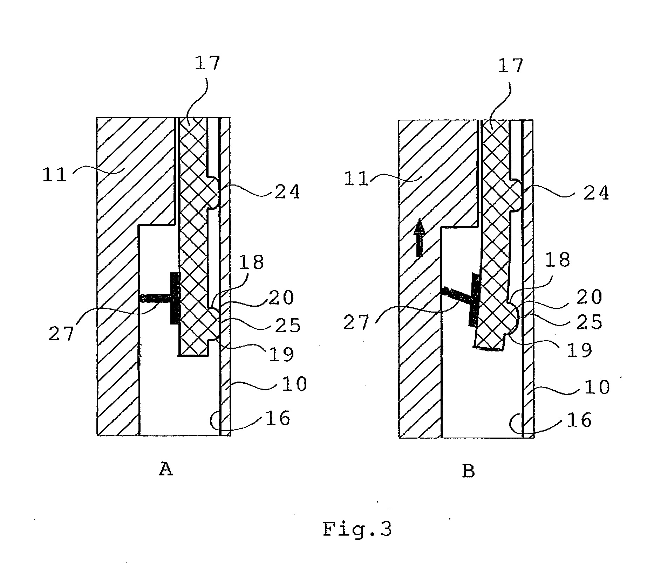

Bei der Ausführungsform der Fig. 3 zeigt ebenfalls Darstellung A die Spritze im Ruhezustand und Darstellung B die Spritze nach der Anfangsbewegung. Das Dichtelement 17 ist wiederum so gestaltet, dass es von selbst mit einem ausreichenden Anlagedruck an der Innenwand 16 des Zylinders 10 anliegt. Das Dichtelement 17 liegt in diesem Beispiel nicht über seine gesamte Länge an der Innenwand 16 an, sondern der Kontakt ist auf zwei separate Kontaktbereiche 24, 25 beschränkt. Der Kontaktbereich 25 hat ein vorderes Ende 18, ein hinteres Ende 19, sowie einen Frontabschnitt 20.

Zwischen dem Dichtelement 17 und dem Kolben 11 erstreckt sich ein Übertragungselement 27, das dazu ausgelegt ist, das Dichtelement 17 so nach innen zu ziehen, dass sich der Anlagedruck im Kontaktbereich 25 und insbesondere im Frontabschnitt 20 des Kontaktbereichs 25 vermindert. Je nach Auslegung kann der Kontaktbereich 25 vollständig von der Innenwand abgehoben werden. Wenn kein Druck mehr auf den Kolben 11 ausgeübt wird, kehrt das Dichtelement 17 aus dem elastisch verformten Zustand (Darstellung B) in den Ausgangszustand zurück und bringt damit über das Übertragungselement 27 auch den Kolben 11 wieder in den Ruhezustand. Bei dieser Ausführungsform findet eine Reduzierung des Anlagedrucks nur im hinteren Kontaktbereich 25 statt, während der Anlagedruck im vorderen Kontaktbereich 24 durch die Anfangsbewegung nicht verändert wird.

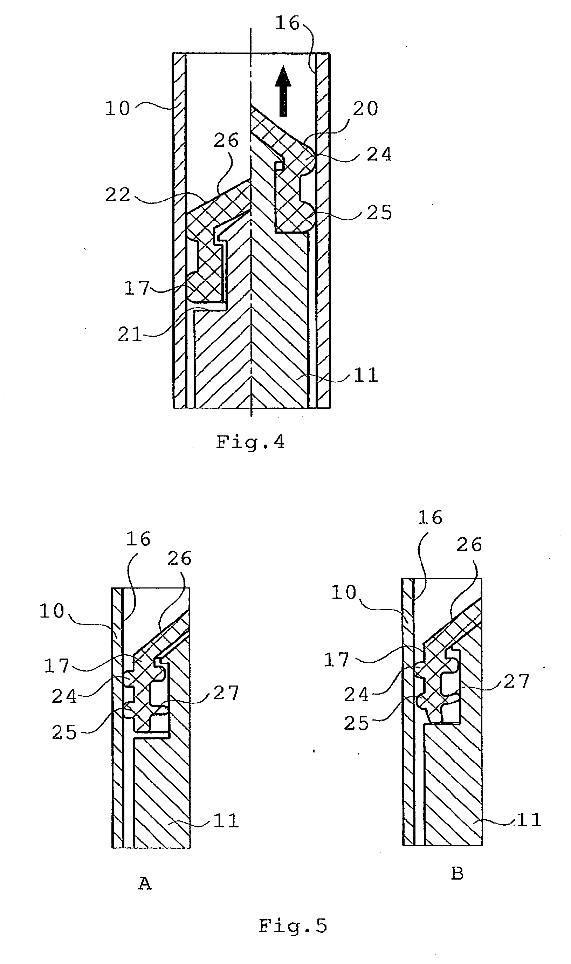

In der zweigeteilten Darstellung der Fig. 4 ist in der linken Hälfte die Spritze im Ruhezustand und in der rechten Hälfte die Spritze bei einer über die Anfangsbewegung hinaus fortgesetzten Bewegung gezeigt. Das Dichtelement 17 erstreckt sich um den Kolben 11 herum und weist zwei voneinander getrennte Kontaktbereiche 24, 25 auf. Das Dichtelement 17 ist so geformt, dass die Kontaktbereiche 24, 25 von selbst mit einem ausreichenden Anlagedruck an der Innenwand 16 des Zylinders 10 anliegen. Einstückig an das Dichtelement 17 angeschlossen ist eine kegelförmige Kappe 26, die das vordere Ende des Kolbens 11 bedeckt. Durch die geschlossene Kappe 26 wird erreicht, dass es außer dem Kontaktbereich zwischen dem Dichtelement 17 und dem Zylinder 10 keine weiteren Austrittspfade gibt. Im Ruhezustand liegt die Spitze des Kolbens 11 an der Kappe 26 des Dichtelements 17 an. Abgesehen von der Spitze des Kolbens 11 besteht ein Freiraum zwischen dem Kolben 11 und dem Dichtelement 17. Dies gilt sowohl für den äußeren Bereich der Kappe 26 als auch in Radialrichtung als auch für den Abstand zwischen der Schulter 21 des Kolbens 11 und dem hinteren Ende des Dichtelements 17.

Wenn der Kolben 11 eine Anfangsbewegung nach vorne vollführt, folgt die Spitze der Kappe 26 dieser Bewegung, so dass die Kappe 26 als Übertragungselement 22 wirkt und das Dichtelement 17 im Kontaktbereich 24 nach innen zieht. Der Anlagedruck im Frontabschnitt 20 des Kontaktbereichs 24 vermindert sich dadurch. Bei der Bewegung nach innen nutzt das Dichtelement 17 den im Ruhezustand bestehenden Freiraum zwischen dem Dichtelement 17 und dem Kolben 11. Die Kappe 26 mit dem Dichtelement 17 bildet ein elastisches Element, das durch die Anfangsbewegung unter Spannung gesetzt wird.

In Fig. 5 ist eine weitere Ausführungsform einer erfindungsgemäßen Spritze dargestellt, wobei Darstellung A den Ruhezustand und Darstellung B den Zustand nach der Anfangsbewegung des Kolbens 11 zeigt. Diese Ausführungsform kombiniert die Merkmale der Figuren 3 und 4. Die Kappe 26 wirkt als Übertragungselement, mit dem der Anlagedruck im Frontabschnitt des Kontaktbereichs 24 vermindert wird. Ein weiteres Übertragungselement 27 erstreckt sich zwischen dem Dichtelement 17 und dem Kolben 11, mit dem der Anlagedruck im Frontabschnitt des Kontaktbereichs 25 über eine Zugkraft wie in Fig. 3 vermindert wird.

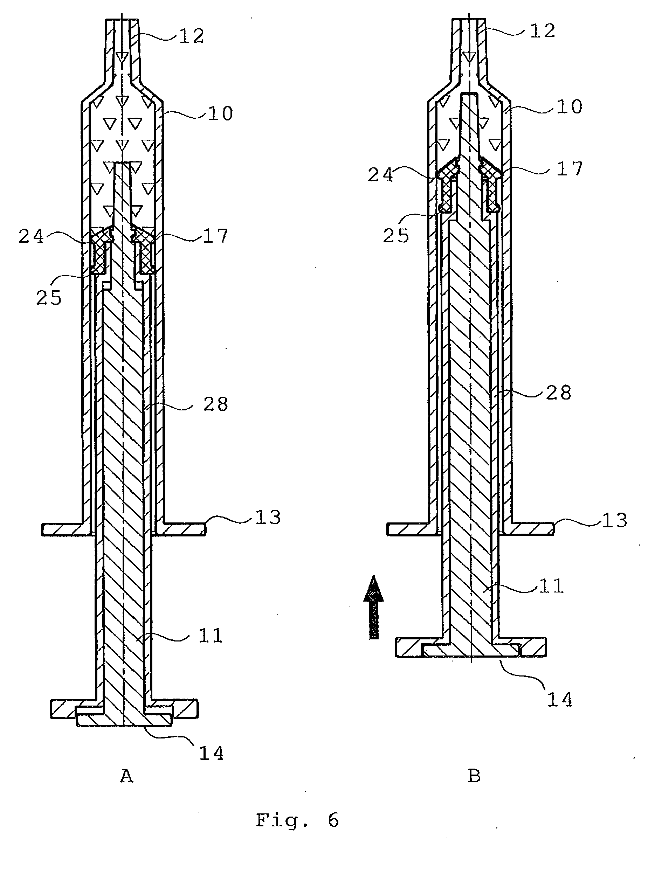

Bei der Ausführungsform der Fig. 6 umfasst die Spritze eine Hülse 28, die in dem Ringspalt zwischen dem Kolben 11 und dem Zylinder 10 angeordnet ist. Die Hülse 28 dient der Führung des Dichtelements 17. Wenn der Kolben 11 eine Anfangsbewegung durchführt, bewegt sich die Hülse 28 nicht, sondern bleibt wie das Dichtelement 17 zunächst an ihrem Ort. Anders als in Fig. 4 bildet der vordere Teil des Dichtelements 17 keine geschlossene Kappe, sondern es besteht ein Durchbruch, durch den sich das vordere Ende des Kolbens 11 hindurch erstreckt. Das vordere Ende des Kolbens 11 ist vorzugsweise an die Form des Auslasses 12 angepasst, so dass eine vollständige Entleerung der Spritze möglich ist.

Das vordere Ende des Dichtelements 17 ist mit dem Kolben 11 verbunden, so dass es der Anfangsbewegung des Kolbens 11 folgt. Wie in Fig. 4 überträgt sich diese Bewegung als Zugkraft auf das Dichtelement 17, so dass der Anlagedruck im vorderen Teil des Kontaktbereichs 24 vermindert wird. Bei einer über die Anfangsbewegung hinaus fortgesetzten Bewegung des Kolbens 11 folgen sowohl das Dichtelement 17 als auch die Hülse 28 der Bewegung des Kolbens 11. Wird kein Druck mehr auf die Druckfläche 14 ausgeübt, endet die Bewegung des Kolbens 11. Das Dichtelement 17 kehrt aus seinem elastisch verformten Zustand in den Ausgangszustand zurück und bewegt damit auch den Kolben 11 wieder in die Position gemäß Darstellung A. Bei dieser Ausführungsform ist die Formveränderung des Dichtelements genau definiert und begrenzt, da der Kolben 11 in der Hülse 28 nur einen bestimmten Weg zurücklegen kann.

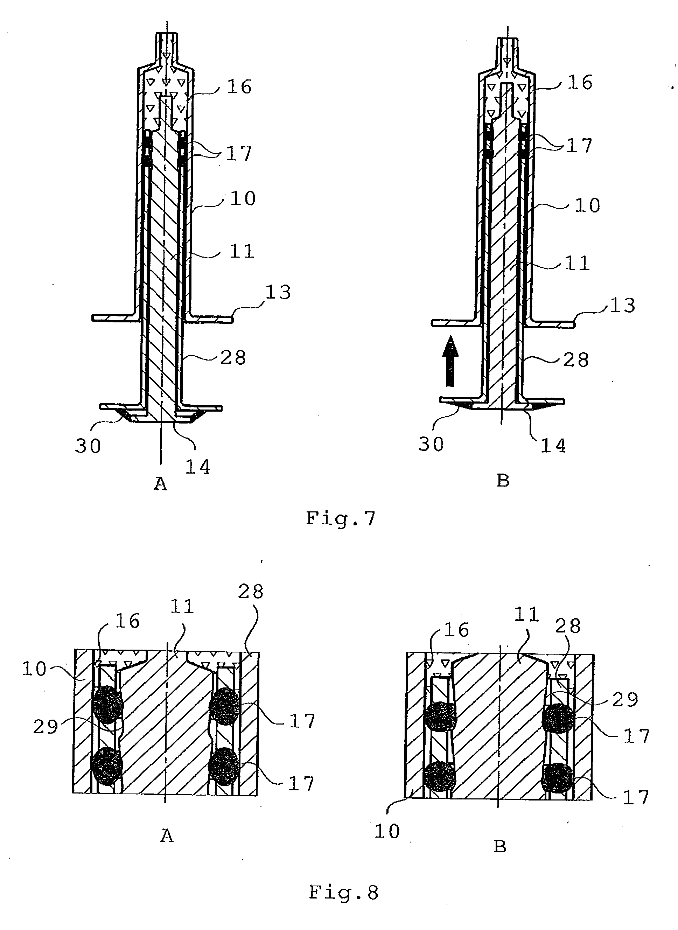

In der Ausführungsform der Fig. 7 umfasst die Spritze zwei Dichtelemente 17, die wiederum durch eine Hülse 28 geführt sind. Der Kolben 11 ist, wie die vergrößerte Darstellung in Fig. 8 zeigt, so geformt, dass er im Ruhezustand (Darstellung A) eine radial nach außen gerichtete Kraft auf die Dichtelemente 17 ausübt. Aufgrund dieser Kraft liegen die Dichtelemente 17 im Ruhezustand mit einem ausreichenden Anlagedruck an der Innenwand 16 des Zylinders 10 an.

Bei einer Anfangsbewegung des Kolbens 11 bewegen sich die Dichtelemente 17 entlang einer Konusfläche 29 des Kolbens 11, so dass sich der Anlagedruck im Kontaktbereich zwischen dem Dichtring und dem Zylinder und insbesondere im Frontabschnitt des Kontaktbereichs vermindert. Der Kolben 11 ist über ein Federelement 30 mit der Hülse 28 verbunden. Das Federelement 30 wird bei der Anfangsbewegung des Kolbens 11 gespannt. Wenn kein Druck mehr auf den Kolben 11 ausgeübt wird, entspannt sich das Federelement 30 wieder und zieht den Kolben 11 relativ zu dem Dichtelement 17 und der Hülse 28 zurück in den Ruhezustand gemäß Darstellung A. Damit wird der ausreichende Anlagedruck zwischen den Dichtelementen 17 und der Innenwand 16 des Zylinders 10 wieder aufgebaut. Aus diesem Ruhezustand heraus kann der Kolben 11 eine erneute Anfangsbewegung durchführen, mit der der Anlagedruck zwischen den Dichtelementen 17 und der Innenwand 16 vermindert wird.

## Patentansprüche

1. Spritze mit einem Zylinder (10), mit einem in dem Zylinder (10) angeordneten Kolben (11) und mit einem Dichtelement (17), das mit einem Kontaktbereich (24, 25, 31) an einer Innenwand (16) des Zylinders (10) anliegt und einen Ringspalt (25) zwischen dem Kolben (11) und der Innenwand (16) des Zylinders (10) abdichtet, wobei der Kontaktbereich (24, 25, 31) einen Frontabschnitt (20) aufweist, der im Ruhezustand mit einem Anlagedruck an der Innenwand (16) des Zylinders (10) anliegt, und wobei der Kolben (11) so mit dem Dichtelement (17) verbunden ist, dass das Dichtelement (17) einer Anfangsbewegung des Kolbens (11) relativ zum Zylinder (10) in Richtung des Auslassendes der Spritze aus dem Ruhezustand heraus nicht folgt und dass das Dichtelement (17) einer fortgesetzten Bewegung des Kolbens (11) relativ zum Zylinder (10) in Richtung des Auslassendes der Spritze folgt, **dadurch gekennzeichnet, dass** sich bei der Anfangsbewegung der Anlagedruck zwischen dem Frontabschnitt (20) des Kontaktbereichs und der Innenwand (16) vermindert, ohne dass der Kontaktbereich (24, 25, 31) vollständig von der Innenwand (16) abgehoben wird.

2. Spritze nach Anspruch 1, **dadurch gekennzeichnet, dass** ein Übertragungselement (22, 27) zwischen dem Kolben (11) und dem Dichtelement (17) angeordnet ist und dass das Übertragungselement (22, 27) bei der Anfangsbewegung des Kolbens (11) eine Kraft auf das Dichtelement (17) überträgt, mit der der Anlagedruck vermindert wird.

3. Spritze nach Anspruch 2, **dadurch gekennzeichnet, dass** das Übertragungselement (22) zur Übertragung einer Zugkraft auf das Dichtelement (17) ausgebildet ist.

4. Spritze nach einem der Ansprüche 1 bis 3, **dadurch gekennzeichnet, dass** die Haftreibung zwischen dem Kontaktbereich (24, 25, 31) und der Innenwand (16) des Zylinders (10) nach der Anfangsbewegung um mindestens 10%, vorzugsweise um mindestens 20%, weiter vorzugsweise um mindestens 30% geringer ist als im Ruhezustand.

5. Spritze nach einem der Ansprüche 1 bis 4, **dadurch gekennzeichnet, dass** im Ruhezustand der Kolben (11) eine radial nach außen gerichtete Kraft auf das Dichtelement (17) ausübt.

6. Spritze nach Anspruch 5, **dadurch gekennzeichnet, dass** das Dichtelement (17) sich bei der Anfangsbewegung entlang einer Konusfläche (29) des Kolbens (11) bewegt, so dass sich der Anlagedruck vermindert.

7. Spritze nach einem der Ansprüche 1 bis 6, **dadurch gekennzeichnet, dass** im Ruhezustand ein Freiraum zwischen dem Dichtelement (17) und dem Kolben (11) besteht und dass das Dichtelement (17) sich bei der Anfangsbewegung des Kolbens (11) in den Freiraum bewegt.

8. Spritze nach einem der Ansprüche 1 bis 7, **dadurch gekennzeichnet, dass** eine zwischen dem Kolben (11) und dem Zylinder (10) angeordnete Hülse (28) zur Führung des Dichtelements (17) vorgesehen ist.

9. Spritze nach einem der Ansprüche 1 bis 8, **dadurch gekennzeichnet, dass** sie ein elastisches Element umfasst, das mit der Anfangsbewegung des Kolbens (11) gespannt wird.

10. Spritze nach Anspruch 8, **dadurch gekennzeichnet, dass** sie ein elastisches Element umfasst, welches mit der Anfangsbewegung des Kolbens (11) gespannt wird und dass das elastische Element (30) zwischen dem Kolben (11) und der Hülse (28) angeordnet ist.

## Claims

1. A syringe with a cylinder (10), with a plunger (11) arranged within the cylinder (10) and with a sealing element (17), which rests against an inner wall (16) of the cylinder (10) with a contact region (24, 25, 31) and seals an annular gap (25) between the plunger (11) and the inner wall (16) of the cylinder (10), wherein the contact region (24, 25, 31) has a front section (20), which, in the rest state, rests against the inner wall (16) of the cylinder (10) with contact pressure, and wherein the plunger (11) is connected to the sealing element (17) such that the sealing element (17) does not follow an initial movement of the plunger (11) relative to the cylinder in the direction of the outlet end of the syringe from the rest state and that the sealing element (17) follows a continued movement of the plunger (11) relative to the cylinder in the direction of the outlet end of the syringe, **characterized in that** the contact pressure between the front section (20) of the contact region and the inner wall (16) is reduced during the initial movement, without the contact region (24, 25, 31) being completely lifted off from the inner wall (16).

2. The syringe as claimed in claim 1, **characterized in that** a transmission element (22, 27) is arranged between the plunger (11) and the sealing element (17) and **in that** the transmission element (22, 27) transmits a force onto the sealing element (17) during the initial movement of the plunger (11), said force reducing the contact pressure.

3. The syringe as claimed in claim 2, **characterized in that** the transmission element (22) is designed to transmit a tensile force onto the sealing element (17).

4. The syringe as claimed in one of claims 1 to 3, **characterized in that** the static friction between the contact region (24, 25, 31) and the inner wall (16) of the cylinder (10) after the initial movement has reduced by at least 10%, preferably by at least 20%, more preferably by at least 30% compared to the rest state.

5. The syringe as claimed in one of claims 1 to 4, **characterized in that** a radially outwardly directed force acts on the sealing element (17) in the rest state of the plunger (11).

6. The syringe as claimed in claim 5, **characterized in that**, during the initial movement, the sealing element (17) moves along a conical surface (29) of the plunger (11) such that the contact pressure reduces.

7. The syringe as claimed in one of claims 1 to 6, **characterized in that**, in the rest state, there is a clearance between the sealing element (17) and the plunger (11) and **in that** the sealing element (17) moves into the clearance during the initial movement of the plunger (11).

8. The syringe as claimed in one of claims 1 to 7, **characterized in that** a sleeve (28) arranged between the plunger (11) and the cylinder (10) is provided for guiding the sealing element (17).

9. The syringe as claimed in one of claims 1 to 8, **characterized in that** it comprises an elastic element which is tensioned with the initial movement of the plunger (11).

10. The syringe as claimed in claim 8, **characterized in that** it comprises an elastic element which is tensioned with the initial movement of the plunger (11) and **in that** the elastic element (30) is arranged between the plunger (11) and the sleeve (28).

## Revendications

1. Seringue, comportant un cylindre (10), un piston (11), disposé dans le cylindre (10), et un élément d'étanchéité (17) qui est en appui avec une zone de contact (24, 25, 31) contre une paroi intérieure (16) du cylindre (10) et assure l'étanchéité d'une fente annulaire (25) entre le piston (11) et la paroi intérieure (16) du cylindre (10), la zone de contact (24, 25, 31) comportant une partie frontale (20) qui, en position de repos, est en appui avec une pression d'appui contre la paroi intérieure (16) du cylindre (10), et le piston (11) étant relié à l'élément d'étanchéité (17) de telle sorte que l'élément d'étanchéité (17) ne suit pas un mouvement initial du piston (11) par rapport au cylindre (10) vers l'extrémité de sortie de la seringue pour sortir de la position de repos et de telle sorte que l'élément d'étanchéité (17) suit un mouvement consécutif du piston (11) par rapport au cylindre (10) vers l'extrémité de sortie de la seringue, **caractérisée en ce que** pendant le mouvement initial, la pression d'appui entre la partie frontale (20) de la zone de contact et la paroi intérieure (16) diminue sans que la zone de contact (24, 25, 31) soit complètement détachée de la paroi intérieure (16).

2. Seringue selon la revendication 1, **caractérisée en ce qu'**un élément de transmission (22, 27) est disposé entre le piston (11) et l'élément d'étanchéité (17) et **en ce que**, au moment du mouvement initial du piston (11), ledit élément de transmission (22, 27) transmet sur l'élément d'étanchéité (17) une force qui induit la diminution de la pression d'appui.

3. Seringue selon la revendication 2, **caractérisée en ce que** l'élément de transmission (22, 27) est réalisé pour transmettre une force de traction sur l'élément d'étanchéité (17).

4. Seringue selon l'une quelconque des revendications 1 à 3, **caractérisée en ce que** le frottement statique entre la zone de contact (24, 25, 31) et la paroi intérieure (16) du cylindre (10) après le mouvement initial est inférieur d'au moins 10 %, de préférence d'au moins 20 %, encore mieux d'au moins 30 % à celui dans la position de repos.

5. Seringue selon l'une quelconque des revendications 1 à 4, **caractérisée en ce que** dans la position de repos, le piston (11) exerce sur l'élément d'étanchéité (17) une force dirigée radialement vers l'extérieur.

6. Seringue selon la revendication 5, **caractérisée en ce que**, pendant le mouvement initial, l'élément d'étanchéité (17) se déplace le long d'une surface conique (29) du piston (11), de telle sorte que la pression d'appui diminue.

7. Seringue selon l'une quelconque des revendications 1 à 6, **caractérisée en ce que**, dans la position de repos, il existe un dégagement entre l'élément d'étanchéité (17) et le piston (11) et **en ce que** l'élément d'étanchéité (17) se déplace dans ledit dégagement pendant le mouvement initial du piston (11).

8. Seringue selon l'une quelconque des revendications 1 à 7, **caractérisée en ce qu'**entre le piston (11) et le cylindre (10) est prévue une gaine (28) pour le guidage de l'élément d'étanchéité (17).

9. Seringue selon l'une quelconque des revendications 1 à 8, **caractérisée en ce qu'**elle comporte un élément élastique qui est tendu par le mouvement initial du piston (11).

10. Seringue selon la revendication 8, **caractérisée en ce qu'**elle comporte un élément élastique qui est tendu par le mouvement initial du piston (11) et **en ce que** l'élément élastique (30) est disposé entre le piston (11) et la gaine (28).
